# EUROPEAN PATENT SPECIFICATION

(11) **EP 2 435 826 B1**
(45) Date of publication and mention of the grant of the patent: **21.05.2014**
(21) Application number: 09779547.0
(22) Date of filing: 26.05.2009
(51) Int. Cl.: G01N 33/50

(54) **URINARY GM2 ACTIVATOR PROTEIN AS A MARKER OF ACUTE RENAL FAILURE OR THE RISK OF DEVELOPING ACUTE RENAL FAILURE**
URIN GM2-AKTIVATOR PROTEIN ALS MARKER VON AKUTEM NIERENVERSAGEN ODER NIERENVERSAGENRISIKO
PROTÉINE ACTIVATRICE DE GM2 URINAIRE EN TANT QUE MARQUEUR DE L'INSUFFISANCE RÉNALE AIGUË OU DU RISQUE DE DÉVELOPPER UNE INSUFFISANCE RÉNALE AIGUË

(43) Date of publication of application: 04.04.2012
(73) Proprietor: UNIVERSIDAD DE SALAMANCA, 37008 Salamanca (ES)
(72) Inventor: QUIROS LUIS, Yaremi, E-37008 Salamanca (ES); FERREIRA REDONDO, Laura, E-37008 Salamanca (ES); SANCHO MARTÍNEZ, Sandra María, E-37008 Salamanca (ES); GONZALEZ DE BUITRAGO ARRIERO, José Manuel, E-37008 Salamanca (ES); LOPEZ HERNANDEZ, Francisco José, E-37008 Salamanca (ES); LOPEZ NOVOA, José Miguel, E-37008 Salamanca (ES)
(74) Representative: Pons Ariño, Angel
(86) International application number: PCT/EP2009/056381
(87) International publication number: WO 2010/136059

(56) References cited:
- US-A1- 2007 065 881
- BANERJEE, A. ET AL: "Enzyme-linked immunosorbent assay for the ganglioside GM2-activator protein" Z. PHYSIOL.CHEM., vol. 365, March 1984 (1984-03), pages 347-356, XP002551399
- DEHNE MARIUS G ET AL: "Evaluation of sE-Selectin and sICAM-1 as parameters for renal function" RENAL FAILURE, NEW YORK, US, vol. 30, no. 7, 1 January 2008 (2008-01-01), pages 675-684, XP009124080 ISSN: 0886-022X

### SEQUENCE LISTING

<110> Universidad de Salamanca
<120> Urinary GM2 activator protein as a marker of acute renal failure or the risk of developing acute renal failure
<130> PCT1367.35
<160> 2
<170> PatentIn version 3.5
<210> 1
   <211> 189
   <212> PRT
   <213> Homo sapiens
<400> 1
   Met Gln Ala Pro Leu Leu Ile Ala Leu Gly Leu Leu Leu Ala Thr Pro
   1               5                   10                  15
   Ala Gln Ala His Leu Lys Lys Pro Ser Gln Leu Ser Ser Phe Ser Trp
               20                  25                  30
   Asp Asn Cys Asp Glu Gly Lys Asp Pro Ala Val Ile Arg Ser Leu Thr
           35                  40                  45
   Leu Glu Pro Asp Pro Ile Val Val Pro Gly Asn Val Thr Leu Ser Val
       50                  55                  60
   Val Gly Ser Thr Ser Val Pro Leu Ser Ser Pro Leu Lys Val Asp Leu
   65                  70                  75                  80
   Val Leu Glu Lys Glu Val Ala Gly Leu Trp Ile Lys Ile Pro Cys Thr
                   85                  90                  95
   Asp Tyr Ile Gly Ser Cys Thr Phe Glu His Phe Cys Asp Val Leu Asp
               100                 105                 110
   Met Leu Ile Pro Thr Gly Glu Pro Cys Pro Glu Pro Leu Arg Thr Tyr
           115                 120                 125
   Gly Leu Pro Cys His Cys Pro Phe Lys Glu Gly Thr Tyr Ser Leu Pro
       130                 135                 140
   Lys Ser Glu Phe Val Val Pro Asp Leu Glu Leu Pro Ser Trp Leu Thr
   145                 150                 155                 160
   Thr Gly Asn Tyr Arg Ile Glu Ser Val Leu Ser Ser Ser Gly Lys Arg
                      165                 170                 175
   Leu Gly Cys Ile Lys Ile Ala Ala Ser Leu Lys Gly Ile
               180                 185
<210> 2
   <211> 14
   <212> PRT
   <213> Homo sapiens
<400> 2
   Ser Trp Asp Asn Cys Asp Glu Gly Lys Asp Pro Ala Val Ile
   1               5                   10

## Description

The invention relates to a method of determining the risk of developing acute renal failure (ARF) in an individual, or of determining an ARF, and a method of predicting the progression of an ARF, by detecting and/or quantifying the protein Ganglioside GM2 Activator Protein (GM2AP). The failure can be due to the administration of at least one nephrotoxic agent, wherein the nephrotoxic agent can be an aminoglycoside antibiotic as for example gentamicin.

### BACKGROUND ART

Acute renal failure (ARF) is an extremely serious condition resulting from an abrupt loss of the kidney's excretory function, sufficient to prevent blood clearing of waste products and water, and electrolytic balance (Bellomo R, Kellum JA and Ronco C. 2007, Intensive Care Med. 33: 409-13). ARF can be induced by a wide variety of insults including drugs, chemical toxins, hypoxia, obstruction of the urinary ways, infections, and others (Binswanger U. 1997, Kidney Blood Press Res., 20: 163). ARF poses an enormous human and socio-economic burden derived from its high incidence and mortality rate. It is estimated that nearly 1% of hospital admissions are associated to ARF, and about 2-7% of hospitalized patients eventually develop ARF (Kellum JA and Hoste EA. 2008, Scand J Clin Lab Invest Suppl., 241:6-11).

A key determinant for a more successful clinical handling of ARF is very early diagnosis, when damage is still as mild as possible, which greatly improves the efficacy of insult retrieval (when possible), therapeutic intervention and patient's outcome. As such, the identification of earlier and more sensitive biomarkers is an obvious goal. In clinical practice, ARF is still diagnosed when renal dysfunction gives rise to measurable symptoms. This typically relies on the determination of creatinine and urea levels in the blood. Their serum concentration most commonly increases as GFR (Glomerular Filtration Rate) decreases. However, at this stage, ARF becomes difficult to handle. As such, present diagnostic tendencies aim at detecting incipient pathophysiological events occurring at early stages, when damage is less extensive (Vaidya VS, Ferguson MA and Bonventre JV. 2008, Rev Pharmacol Toxicol., 48:463-93). Among them, measurement of certain cellular enzymes present in the urine as a consequence of tubular cell rupture is presently the finest method for an early detection of ARF coursing with tubular damage. These enzymes include N-acetyl-beta-D-glucosaminidase (NAG), but also others such as lactate dehydrogenase (DHL), alkaline phosphatase (ALP) or gamma-glutamyl transpeptidase (GGT). Most of these enzymes have a moderate value as early and sensitive urinary markers for ARF, mostly due to problems of stability and inhibition by other components in the urine (Vaidya VS, Ferguson MA and Bonventre JV., 2008, Rev Pharmacol Toxicol., 48:463-93). By far, NAG is the best characterized as a subtle marker of renal damage, although it is still rarely used as a routine analysis parameter. New urine markers are currently in an advanced degree of validation for the very early diagnosis and prognosis of ARF. They include kidney injury molecule-1 (KIM-1), neutrophil gelatinase-associated lipocalin (NGAL), plasminogen activator inhibitor-1 (PAI-1), cistatin C, interleukin 18, retinol binding protein (RBP) and others.

Another important aspect related to ARF is the action of certain potentially nephrotoxic drugs, at subtoxic doses, to predispose or sensitize individuals to undergo an ARF more easily in response to other nephrotoxins. A typical clinical situation exists where a patient treated with a drug (e.g. gentamicin) showing no signs of renal disease is subject or exposed concomitantly or subsequently to another potentially nephrotoxic agent, such as another drug, a diagnostic contrast, a heavy metal, etc., also within a theoretically subtoxic regime. If this is so, subtoxic treatments with potential nephroxins would pose relevant clinical situations of special importance due to their hidden nature, which should be addressed from the diagnostic and therapeutic perspectives. Nowadays, renal failure is diagnosed using different parameters. For example, there is a consensus criterion for the diagnosis of acute renal failure (ARF) that establishes different degrees of the disease. This consensus criterion, named with the acronym RIFLE (Ricci Z, Cruz D and Ronco C. 2008, Kidney Int., 73: 538-46) is based on serum creatinine increment, glomerular filtration rate decrease and urine flow decrease. To establish this criterion, it is very important to carry out a large number of tests. The level of creatinine in plasma is used as a marker of renal injury. Healthy kidneys take creatinine out of the blood and put it into the urine to leave the body. When the kidneys are not working well, creatinine builds up in the blood but values of creatinine are also variable and can be affected by diet. Other markers for diagnosing renal failure are blood urea nitrogen or proteinuria.

Gentamicin is an aminoglycoside antibiotic widely used worldwide against Gram-negative infections. Its therapeutic efficacy and use are severely restricted by its toxicity, which mainly occurs at the renal and auditory levels (Martínez-Salgado C, López-Hernández FJ and López-Novoa JM. 2007, Toxicol Appl Pharmacol., 223: 86-98). Gentamicin-induced nephrotoxicity appears in 10-25% of therapeutic courses (Leehey DJ et al., 1993, J. Am. Soc. Nephrol., 4: 81-90). It is characterized mostly by tubular damage (Nakakuki M et al., 1996, Can J Physiol Pharmacol., 74: 104-11), but glomerular (Martínez-Salgado C, López-Hernández FJ and López-Novoa JM. 2007, Toxicol Appl Pharmacol., 223: 86-98) and vascular (Goto T et al., 2004, Virchows Arch., 444: 362-74; Se 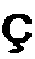 ilmi 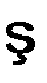 MA, et al., 2005, Nephron Physiol., 100: 13-20) alterations might also appear in a dose-dependent manner (Hishida A et al., 1994, Ren Fail., 16: 109-16).

Ganglioside GM2 activator protein (GM2AP) is a small glycolipid transport protein which acts as a substrate specific co-factor for the lysosomal enzyme β-hexosaminidase A. This enzyme together with GM2AP, catalyzes the degradation of ganglioside GM2 (a sialic acid containing glycosphingolipid), and other molecules containing terminal N-acetyl hexosamines. GM2AP has been related with hepatic diseases using it as a marker for the prognosis of hepatotoxicity of some hepatotoxins (US 7469185) but not with renal diseases or acute renal failure.

The use of any biomarker that is expressed early in acute renal failure or renal damage, can be a useful tool for determining the risk of any patient to suffer this damage. This tool could also be used, among many other possible applications, to modify the administration of any drugs to any individuals in order to prevent the worsening of the health of said individual.

### SUMMARY OF THE INVENTION

The invention relates to a method for determining the risk of developing acute renal failure (ARF) in an individual, or for determining an ARF, and a method of predicting the progression of an ARF, by detecting and/or quantifying the protein Ganglioside GM2 Activator Protein (GM2AP). The failure can be due to the administration of at least one nephrotoxic agent, wherein the nephrotoxic agent can be an aminoglycoside antibiotic as for example gentamicin.

The present invention provides evidence that the predisposition to ARF induced by gentamicin correlates with an increased excretion of the protein GM2AP. The examples further emphasize the usefulness of GM2AP as a potential urinary biomarker of gentamicin-induced predisposition to ARF.

Therefore, the present invention provides tools to improve the detection of an ARF and, in addition, the possibility of determining the risk of developing ARF. A consequence of this provision is the prediction of the progression of an ARF, that is, the monitoring of the progression of the ARF when the individual is treated with, for example, but not limited to, therapeutic substances, or when the individual is exposed to any condition or agent that has clear nephrotoxic effect or not.

The above cited improvement in the detection of an ARF by means of the detection and/or quantification of GM2AP is clear from the methods cited in the background art, as for example, determining the plasma creatinine concentration, blood urea nitrogen (BUN), urinary excretion of proteins such as for example N-acetyl-beta-D-glucosaminidase (NAG). This evidence is shown in the examples.

In addition, the present invention emphasizes several unexpected or surprising effects:
- On the one hand, the detection of GM2AP in urine samples supposes an additional advantage for the patient since the evacuation of this fluid is a necessary physiological fact that occurs normally. This supposes no aggressive sampling in the individual.
- On the other hand, the administration of a subtoxic regime of an aminoglycoside antibiotic (gentamicin) to rats, predisposes individuals treated with a second nephrotoxic agent, for instance uranyl nitrate, to an ARF and this predisposition can be detected by means of the presence of GM2AP in the urine. This fact was demonstrated verifying higher concentrations of creatinine and blood urea nitrogen (BUN) in blood or the urinary protein excretion of rats exposed to these toxins with respect to rats treated with a placebo. In the present invention it is also demonstrated that the co-treatment with both compounds produced an increase of the concentration in plasma creatinine as well as when gentamicin and uranyl nitrate are administered in sequential form, and both administrations separated by a period of rest.
- In addition, protein GM2AP can be detected in a sample of urine of an individual from the first day of treatment with a toxic regime of gentamicin (in the present invention in the rats the toxic dose employed is 150 mg/kg/day) whereas the other markers of renal insufficiency: creatinine, NAG, proteinuria, KIM-1 or PAI-1, are detected at the fourth day in the same urine sample.

Thus, a first aspect of the present invention refers to a method for providing data for determining the risk of developing ARF, or for determining ARF, in an individual comprising detecting and/or quantifying a protein at least 70% or 100% identical to SEQ ID NO: 1, in a biological sample isolated from an individual (subject).

The term "% identical" as is understood in this invention refers to the % of identity between two amino acid sequences. The % of identity is a count of the number of positions over the length of the alignment of two sequences where all of the amino acids at that position are identical.

The protein detected and/or quantified can be at least 70, 75, 80, 85, 90 or 95% identical to SEQ ID NO: 1.

In a preferred embodiment, the protein detected and/or quantified is at least 70% identical to SEQ ID NO: 1. In a more preferred embodiment, the cited protein is SEQ ID NO: 1.

The term ARF as is understood in this invention refers to an acute renal failure in any stage (or severity) of a renal dysfunction, or the term ARF also refers to an acute renal damage. The ARF can be caused by kidney damage or by any damage or disease whose origin can be, for example, but not limited to, genetic, immune, ischemic or treatment with any drug. The kidney damage or any damage or disease mentioned above can be also produced by a surgical procedure, for example, but not limited to, in kidney, prostate, bladder, ureter or urethra.

The method provides data for determining the risk of developing acute renal failure (ARF), or for determining ARF, in an individual. The last stage of the risk of developing an ARF is the ARF, and therefore, the method is useful for both conditions of an ARF in an individual.

The amino acidic sequence SEQ ID NO: 1 is the amino acidic sequence of the protein GM2AP in *Homo sapiens* (humans) accession number CAA43994.

The proteins (amino acidic sequences) with at least 70% of identity to the amino acidic sequence SEQ ID NO: 1, are isoforms or amino acidic sequences that are homologous to SEQ ID NO: 1 in several animals. The method of the present invention can be applied for veterinary purposes. The veterinary purposes can be applied to vertebrate organisms as for example, but not limited to, *Equus caballus, Bos taurus, Felis catus* or *Canis lupus familiaris.* The vertebrate organism can be also a fish species because of interest in fish farming. This percentage of identity has been chosen according to a Blastp from National Center for Biotechnology Information (NCBI) (http://www.ncbi.nlm.nih.gov/) (see Table 1).

Table 1. Percentage of identity of several homologous sequences of SEQ ID NO: 1 from different organisms with respect to said sequence SEQ ID NO: 1 *(Homo sapiens*)*.* The access numbers of the cited sequences used in this table are:
*Homo sapiens* (CAA43994), *Ratus norvegicus* (BAC24018), *Mus musculus* (NP_034429), *Equus caballus* (NP_001075381), *Bos taurus* (XP_580665), *Felis catus* (AAS64350), *Canis lupus familiaris* (XP_536462), *Salmo salar* (ACN11254), *Tribolium castaneum* (XP_973964), *Hydra magnipapillata* (XP_002157755).

| **Species 1** | **Species 2** | **% Identity** |
|---|---|---|
| ***Homo sapiens*** | ***Ratus norvegicus*** | **72** |
| | ***Mus musculus*** | **72** |
| | ***Equus caballus*** | **71** |
| | ***Bos taurus*** | **65** |
| | ***Felis catus*** | **68** |
| | ***Canis lupus familiaris*** | **57** |
| | ***Salmo salar*** | **54** |
| | *Tribolium castaneum (gorgojo)* | 32 |
| | *Hydra magnipapillata* | 30 |

To refer to any of the proteins with at least 70% of identity to the amino acidic sequence SEQ ID NO: 1, or to SEQ ID NO: 1, or any fragment thereof, the term "protein/s of the present invention" or "protein/s of the invention" will be used hereafter.

To detect and/or quantify the protein of the present invention it is sufficient to detect one or more fragments of said protein because the fragment is a constituent of the amino acidic sequence and structure of the protein.

The method refers to the detection and the quantification of the protein of the present invention or to its detection or to its quantification.

The protein of the present invention is the product of the expression of a nucleotide sequence. This nucleotide sequence can be any RNA as for example, but not limited to, messenger RNA (mRNA), or a fragment thereof. This nucleotide sequence can also be complementary DNA (cDNA) or a fragment thereof. The cDNA is DNA complementary to an mRNA or is also the nucleotide sequence comprising exons from a genomic sequence but not introns, that is, the coding sequence. The transcription of both genomic sequence of a gene and its cDNA encode for the same mRNA and, therefore, encode for the same protein. In the present invention is also possible to detect any RNA or any DNA, or a fragment thereof, instead of the protein or at the same time.

Another preferred embodiment refers to the previous method that comprises, in addition, a step to compare the data obtained with standard values, to find any significant deviation. The term "standard values" as used herein refers, for instance, but not limited, to data about detecting and/or quantifying the protein of the present invention, or a fragment thereof, in a biological sample obtained from an individual that does not develop an ARF.

The term "significant deviation" as used herein refers to the presence of the protein in the isolated sample or a higher concentration of the protein of the invention in the isolated sample with respect to a sample from a healthy individual, that is, a negative control for renal disease. The healthy individual is determined by means of the measurement of the level of one or more common markers of renal disease. The common marker is, but not limited to, creatinine, blood urea nitrogen or proteinuria.

In another preferred embodiment of the present invention, the method comprises, in addition, the attribution of the significant deviation to the risk of developing ARF, or to developing of an ARF, in the individual. Therefore, this preferred embodiment is a method to diagnose an ARF or to determine the risk of developing acute renal failure (ARF).

The biological sample is a sample isolated from an organism such as the human or animal body that can come from a physiological fluid and/or any cellular tissue of an organism.

The term "risk of developing ARF" as used herein refers to the predisposition of an individual to suffer or develop an ARF. Thus, the method refers to providing useful data for determining if an individual is developing or can develop an ARF. Along with the examples it is demonstrated that the protein GM2AP (SEQ ID NO: 1) can be detected at very early stage of an ARF and this can permit detection of an early ARF as a risk of developing ARF.

In further preferred embodiment of the present invention, the protein of the present invention, can be detected and/or quantified by methods such as, but not limited to, electrophoresis, immunoassay, chromatography and/or microarray technologies. The detection and/or quantification of the protein of the invention can be carried out by means of the combination of any of the previous techniques or any combination thereof. The protein can be detected evaluating its presence or absence. The detection can be carried out by the specific recognition of any fragment thereof by means of any probe and/or any antibody. Also the detected protein of the invention can be quantified so that it serves as reference for comparing these data with standard values to find any significant deviation. This deviation can be interpreted as being a risk of developing acute renal failure or to an ARF itself. In a more preferred embodiment, the protein of the invention, can be detected and/or quantified by means of electrophoresis and/or immunoassay.

Electrophoresis is an analytical technique of separation based on the movement or migration of macro-molecules dissolved in a medium (electrophoresis buffer), through a matrix or a solid support as a result of the action of an electric field. The behaviour of the molecule depends on its electrophoretic mobility and this mobility depends on the charge, size and form. Numerous variations of this technique based on the equipment used, supports and conditions exist in physical chemistry to carry out the separation. The electrophoresis is selected from the list that comprises capillary electrophoresis, electrophoresis in paper, agarose gel electrophoresis, poliacrilamide gel electrophoresis, isoelectric focusing (electrofocusing), or bidimensional electrophoresis.

An immunoassay is a biochemical test that measures the concentration of a substance in a biological liquid using the reaction of an antibody or antibodies to its antigen. The assay takes advantage of the specific binding of an antibody to its antigen. Detecting the quantity of antibody or antigen can be achieved by a variety of methods. One of the most common is to label either the antigen or antibody. The label may comprise, but is not limited to, an enzyme, radioisotopes (radioimmunoassay), magnetic labels (magnetic immunoassay) or fluorescence, and also other techniques including agglutination, nephelometry, turbidimetry or Western Blot. Heterogeneous immunoassays can be competitive or non-competitive. The immunoassay can be competitive: the response will be inversely proportional to the concentration of antigen in the sample, or can be non-competitive (also referred to as the "sandwich assay"): the results are directly proportional to the concentration of the antigen. An immunoassay technique that can be used in the present invention is the Enzyme-Linked ImmunoSorbent Assay (ELISA).

By means of chromatographic techniques, molecules can be separated based on their charges, sizes or molecular masses, through their polarity or their redox potential, among others. The chromatographic technique may be, but is not limited to, chromatography of liquids (partition chromatography, adsorption chromatography, exclusion chromatography or ion exchange chromatography), gas chromatography or chromatography of supercritical fluids.

Microarray technologies of the present invention are based, for example, on the fixation in a solid support of a molecule that recognizes the protein of the present invention. The antibody microarray is the most common protein microarray. In this case, antibodies are spotted and fixed onto the protein chip (solid support) and are used as capture molecules to detect proteins from, but not limited to, biological sample, cell lysates, serum or urine. The term "solid support" as used herein refers to a great variety of materials, for example, ionexchange resin or adsorption, glass, plastic, latex, nylon, gel, esters of cellulose, paramagnetic spheres or the combination of some of them, but is not limited to the previously mentioned materials.

In another preferred embodiment, the biological sample of any of the methods of the present invention is a bodily fluid. The bodily fluid may include fluids that are excreted or secreted from the animal body as well as fluids that normally are not. The bodily fluid may be, but is not limited to, amniotic fluid surrounding a foetus, aqueous humour, blood, blood plasma, interstitial fluid, lymph, breast milk, mucus (including nasal drainage and phlegm), saliva, sebum (skin oil), serum, sweat, tears or urine. The protein of the present invention can be in any biological compartment present in the mentioned bodily fluid as for example, but not limited to, cell or vesicle. In a more preferred embodiment, the bodily fluid is urine.

A further preferred embodiment of the present invention refers to the method wherein the risk of developing ARF, or the ARF, is due to the administration of or exposure to at least one nephrotoxic agent. This nephrotoxic agent can cause one or more renal pathologies due to the level of administration or exposure that may be over a long period of time or limited to a single event, and it may be due to a single or to multiple compounds. The circumstances of exposure may be inadvertent, accidental, intentional overdose or therapeutic necessity (administration). Kidney is the major organ of excretion and because it maintains homeostasis for water-soluble molecules, it can concentrate certain substances actively. In general, the proximal and distal tubules and urothelia can be repaired, but the glomeruli and medulla may have a significantly lower repair facility.

The nephrotoxic agent, when is administered, can be a pharmaceutical composition (therapeutic substance) or, but not limited to, halogenated anaesthetic or a compound that is included in a functional food, or in a vitamin complement, or in a nutritional complement. The nephrotoxic agent, when the individual is exposed, can be in addition, a chemical such as, but not limited to, heavy metal, pesticide or antimicrobial. The pesticide may be, but is not limited to, a fungicide, a herbicide, an insecticide, an algicide, a molluscicide, a miticide or a rodenticide. The insecticide may be, but not limited to, a germicide, an antibiotic, an antibacterial, an antiviral, an antifungal, an antiprotozoal or an antiparasitic agent.

In a further preferred embodiment of any of the methods of the present invention, the nephrotoxic agent is an aminoglycoside antibiotic. Aminoglycosides work, for example, by binding to the bacterial 30S or 50S ribosomal subunits, inhibiting the translocation of the peptidyl-tRNA and also causing misreading of mRNA, leaving the bacterium unable to synthesize proteins vital to its growth. The aminoglycoside antibiotic may be, but is not limited to, amikacin, arbekacin, gentamicin, kanamycin, neomycin, netilmicin, paromomycin, rhodostreptomycin, streptomycin, tobramycin, apramycin, spectinomycin, hygromycin B, verdamicin, astromicin or puromicin. In a more preferred embodiment, the aminoglycoside antibiotic is gentamicin.

Gentamicin is a broad-spectrum aminoglycoside antibiotic that is commonly used to treat infections of aerobic Gram-negative bacteria. Aminoglycosides are absorbed poorly with oral administration, but are excreted rapidly by the kidneys. On the other hand, aminoglycosides diffuse into bacterial cells through porin channels in the outer membrane and are then transported across the cytoplasm. As a result, gentamicin acts by interfering with bacterial protein synthesis but gentamicin can cause kidney damage on the proximal convoluted tubule, particularly in the S1 and S2 segments, which can develop into ARF.

As demonstrated in the present invention, an ARF can be due, as well, to the administration of a second nephrotoxic agent simultaneously or sequentially. This second compound can be uranyl nitrate. As cited in the examples, a subtoxic regime with gentamicin, predisposes an individual to the development of an ARF because when a second nephrotoxic agent (as for example uranyl nitrate) is administered in a subtoxic dose, a renal toxic effect occurs. This renal toxic effect is demonstrated just by adding a single dose of uranyl nitrate, compared with a single dose of a saline solution, immediately after a single dose of gentamicin. This nephrotoxic effect is demonstrated by measuring plasma creatinine concentration, blood urea nitrogen (BUN), urinary excretion of total proteins or specific protein (N-acetyl-glucosaminidase: NAG), or creatinine clearance.

The term "subtoxic dose" as is understand in the present invention, refers to one or more doses administered in any regime in an individual in such a way that the level of one or more common markers of renal disease do not show significant deviation with respect to the levels of a negative control for renal disease. The common marker may be, but is not limited to, creatinine, blood urea nitrogen or proteinuria.

In the present invention is also demonstrated that co-treatment of the individuals simultaneously with both compounds (gentamicin and uranyl nitrate) causes an increase of the concentration in the plasma creatinine as well as when both compound are administered sequentially, both administrations separated by a period of rest, for example, a week.

The protein of the present invention is also detected in urine when individuals are treated with other nephrotoxic drugs as an anti-tumour agent, for example, but not limited to, cysplatin. The protein of the invention is also detected when the individuals are treated only with uranyl nitrate.

Cisplatin is a broad-spectrum anti-tumour agent that is commonly used to treat tumours of the testicles, ovaries, bladder, skin, head and neck, and lungs. Cisplatin diffuses into cells and functions by interstrand and intrastrand crosslinking of the DNA that is lethal to cells. Uranyl nitrate is a highly nephrotoxic agent that causes severe renal insufficiency and acute tubular necrosis. Other target organs include the liver, lungs or brain.

In another preferred embodiment of the method of the present invention, the protein of the invention is detected after 12 hours since the beginning of the administration or exposure to the nephrotoxic agent. Therefore, the protein of the present invention can be used as an early marker of acute renal failure, mainly, but not limited, when the ARF is due to the administration of at least the aminoglycoside antibiotic gentamicin. In this case, as demonstrated in the examples, when gentamicin was administered to rats during 7 days with 150 mg/kg/day, evolution of plasma creatinine concentration, creatinine clearance, NAG excretion, proteinuria and urinary levels of kidney injury molecule 1 (KIM-1) and plasminogen activator inhibitor 1 (PAI-1), were significantly increased from the fourth day whereas the protein SEQ ID NO: 1 was detected at least three days before toxic effect appearance. In a urine sample from an individual, the appearance of the toxic effect is detected by measuring the parameters cited above.

In another preferred embodiment, the protein of the present invention can be detected from 24 hours since the beginning of the administration or exposure to the nephrotoxic agent. As shown in the examples, after the first 24 hours from the beginning of the administration of gentamicin (150 mg/kg/day), the protein is detected with a sufficiently clear level by Western blot assay.

A second aspect of the present invention refers to a method of predicting the progression of an ARF due to the administration of or exposure to at least one nephrotoxic agent, comprising (a) determining a first concentration of a protein that is at least 70% identical to SEQ ID NO: 1, in a bodily fluid isolated from an individual exposed or not to the nephrotoxic agent, (b) determining a second concentration of the protein of the invention in a bodily fluid isolated from the individual of (a) after determining the first concentration of the cited protein in exposed individual, or after initiation of administration or exposure to the nephrotoxic agent in a non-exposed individual. That is, if the determination of the first concentration is carried out in a sample from a individual exposed to the nephrotoxic agent, the second one is determined after the determination of the first concentration, and if the determination of the first concentration is carried out in a sample from a individual without exposure to the nephrotoxic agent, the second one is determined after the initiation of the administration or exposure to the nephrotoxic agent in a non-exposed individual. Then, said second concentration is compared with said first concentration looking for any significant deviation. The significant deviation can be in the sense of increased or reduced values when said second concentration is compared with said first concentration, or when any significant deviation is compared with a previous determination of the concentration.

In the present invention, the term "prediction of the progression" as used herein refers to a conclusion of the monitoring of the progression of an ARF, that is, the announcement about the progression of this pathology.

A preferred embodiment of the present invention refers to the method of predicting the progression of an ARF due to the administration of or exposure to at least one nephrotoxic agent, wherein the said protein is SEQ ID NO: 1.

In another preferred embodiment of the method of predicting the progression of an ARF, the nephrotoxic agent is an aminoglycoside antibiotic and, in a more preferred embodiment, the aminoglycoside antibiotic is gentamicin.

A further preferred embodiment refers to any of the methods of predicting the progression of an ARF, wherein the bodily fluid is urine.

To refer to any of the methods to provide useful data for determining the risk of developing acute renal failure (ARF), or for determining ARF, or to any of the methods of predicting the progression of an ARF, the term "method/s of the present invention" or "method/s of the invention" will be used.

According to a further preferred embodiment, the individual of the method of the present invention is a human. In spite of the above mentioned, the individual of the method of the invention can be an animal because the cited method can be useful in veterinary purposes.

The cell population that suffers the damage or insult, exposed or not to a compound, may be assayed *in vitro* or *in vivo.* For instance, the cell population of freshly isolated renal cells, in particular rat renal cells. In another assay format, *in vivo* exposure may be accomplished by administration of the nephrotoxic agent to a living animal, for instance a laboratory rat.

A third aspect of the present invention refers to a use of a protein at least 70% identical to SEQ ID NO: 1, as biomarker for determining the risk of developing an ARF, or for determining ARF, or for predicting the progression of an ARF.

This biomarker indicates a change in expression or state of a protein that correlates with the risk or progression of an ARF, or with the susceptibility of the disease to a given treatment, or correlates also with the presence of an ARF in an individual. Once a proposed biomarker has been validated, it can be used to diagnose disease risk, presence of disease in an individual, or to tailor treatments for the disease in an individual, for instance, choices of drug treatment or administration regimes. If a treatment alters the presence or amount detected of the biomarker, which has a direct connection to the risk of suffering an ARF, the biomarker serves as a reporter for modifying any treatment or exposure to any nephrotoxic agent.

A preferred embodiment refers to the use, wherein said protein is SEQ ID NO: 1.

A preferred embodiment refers to the use of the protein of the present invention, wherein the risk of developing ARF, or the ARF, or the progression of an ARF is due to the administration of at least one nephrotoxic agent. In further preferred embodiment, the nephrotoxic agent is an aminoglycoside antibiotic, for instance, the aminoglycoside antibiotic is gentamicin.

A still further aspect to the present invention is a kit to provide useful data for determining the risk of developing ARF in an individual, or for determining ARF (diagnosing an ARF), or for predicting the progression of an ARF in the individual, comprising reagents to carry out any of the method of the present invention. The reagents should permit the detection of the protein of the invention and/or its quantification. This kit also can comprise detection solutions.

A preferred embodiment of the present invention is a kit as described above, wherein the reagent is, at least, one or more probes to recognise a protein at least 70% identical to SEQ ID NO: 1. A probe is a substance normally labelled (but not necessarily) and used to detect, identify and/or quantify the protein of the present invention or any fragment thereof. The probe may be, but is not limited to, an antibody.

A preferred embodiment refers to the kit, wherein the protein is SEQ ID NO: 1.

A further preferred embodiment refers to a kit, wherein the probes are attached to a solid support. This solid support is preferably a gel, for instance, a gel of agarose or poliacrylamide.

In a more preferred embodiment, the probes are antibodies used to recognise the protein of the present invention, or a fragment thereof. The antibodies can be monoclonal or polyclonal. In a still more preferred embodiment, the fragment from the protein that is recognized by the antibodies is SEQ ID NO: 2.

Unless otherwise defined, all technical and scientific terms used herein have the same meaning as commonly understood by one of ordinary skill in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, figures and sequence listing are provided by way of illustration and are not intended to be limiting of the present invention.

### BRIEF DESCRIPTION OF THE FIGURES

In order to complement the description that is being made and in order to help better understanding of the characteristics of the invention and in agreement with some preferred examples, figures have been included where, with illustrative and non-limiting character, the following is represented:
**FIG. 1****. Shows the survival rate (A) and body weight evolution (B)** of rats treated during 7 days with saline (0.9% NaCl, n=19) or gentamicin (50 or 150 mg/kg/day, n=42 and n=15 respectively). Data represent the mean ± standard error.
**FIG. 2****. Shows the characterization of renal function.**
   (A) plasma creatinine concentration, (B) blood urea nitrogen (BUN), (C) daily, urinary excretion of proteins, and (D) daily, urinary excretion of N-acetyl-β-D-glucosaminidase (NAG), of rats treated during 7 days with saline (0.9% NaCl, C) or gentamicin (50 or 150 mg/kg/day, respectively G-50 and G-150). Data represent the mean ± standard error. *Control: n_{crea}=46, n_{BUN}=38, nₚᵣₒₜ=25, n_{NAG}* =*12*; *50 mg*/*kg*/*day gentamicin: n_{crea}=57, n_{BUN}=49, nₚᵣₒₜ=25, n_{NAG} =11; and 150 mg*/*kg*/*day gentamicin: n_{crea}*=*18, n_{BUN}*=*4, nₚᵣₒₜ=7, n_{NAG} =7.* ● *p<0.05 versus control group;* ● *p<0.05 versus G-50 group.*

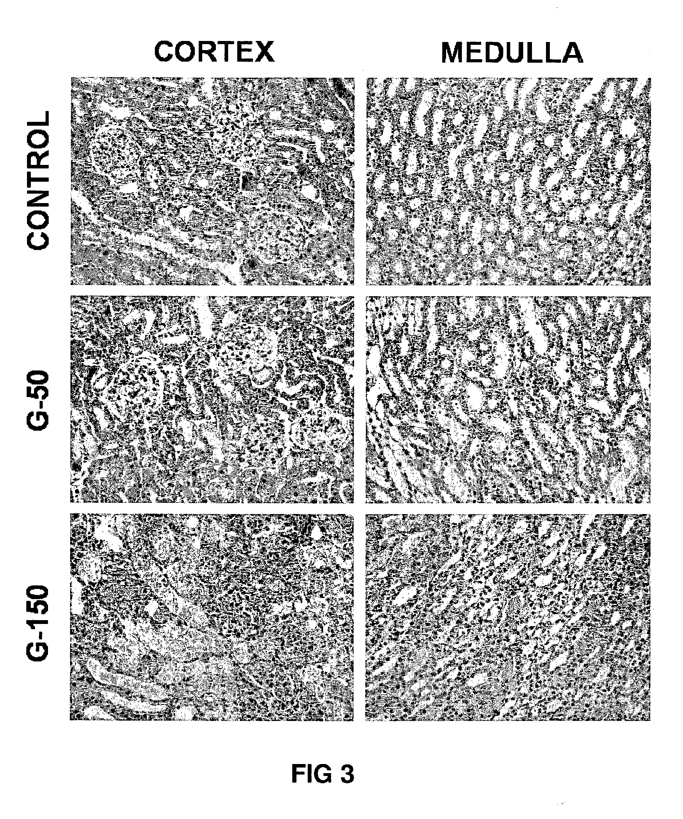

**FIG. 3****. Shows the representative images (100x) of histological slices dyed with haematoxylin and eosin** from the kidneys of rats after 7 days of treatment with physiological saline (Control), gentamicin 50 mg/kg/day (G-50) and 150 mg/kg/day (G-150).

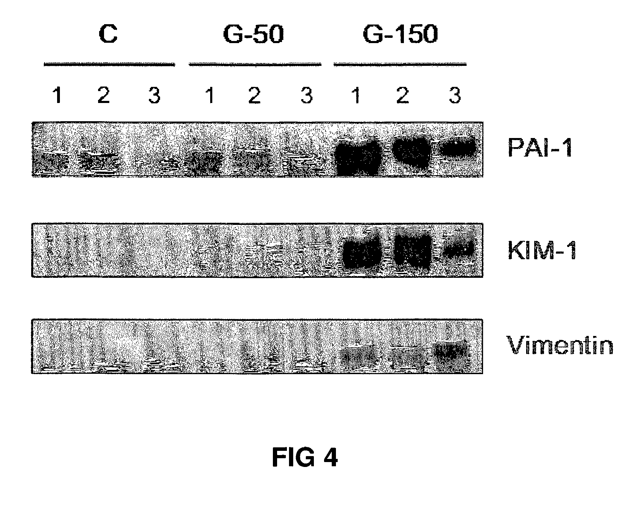

**FIG. 4****. Shows renal expression of markers related to tissue damage and repair.** Representative Western blot images of renal levels of plasminogen activator inhibitor-1 (PAI-1), kidney injury molecule-1 (KIM-1) and vimentin, in renal tissue homogenates from rats treated during 7 days with saline (0.9% NaCl, C) or gentamicin (50 or 150 mg/kg/day, respectively G-50 and G-150). Experiments were performed with samples from three animals (1-3) from each group, randomly selected.

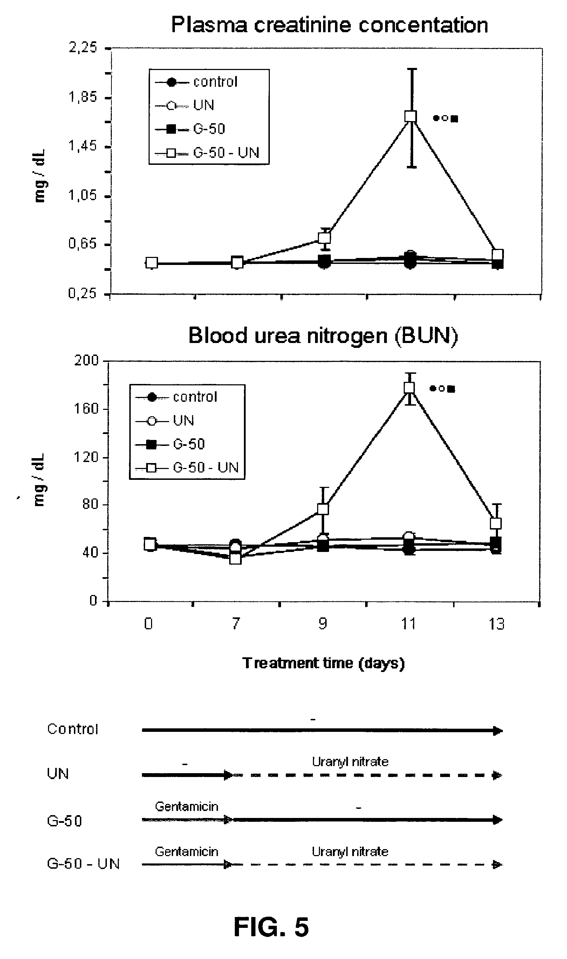

**FIG. 5****. Shows the evolution of plasma creatinine concentration (upper panel) and blood urea nitrogen (BUN) concentration (lower panel)** in rats treated with saline (0.9% NaCl) or gentamicin (50 mg/kg/day) during 7 days, immediately after which they received a single, subtoxic dose of uranyl nitrate (UN, 0.5 mg/kg), or saline (see lower part of the figure). Plasma creatinine and BUN were monitored through the experiment, until six days after UN administration. Data represent the mean ± standard error of samples from 6 animals per group. ● p<0.05 versus Control group; ○ p<0.05 versus UN group; ■ p<0.05 versus G-50 group.

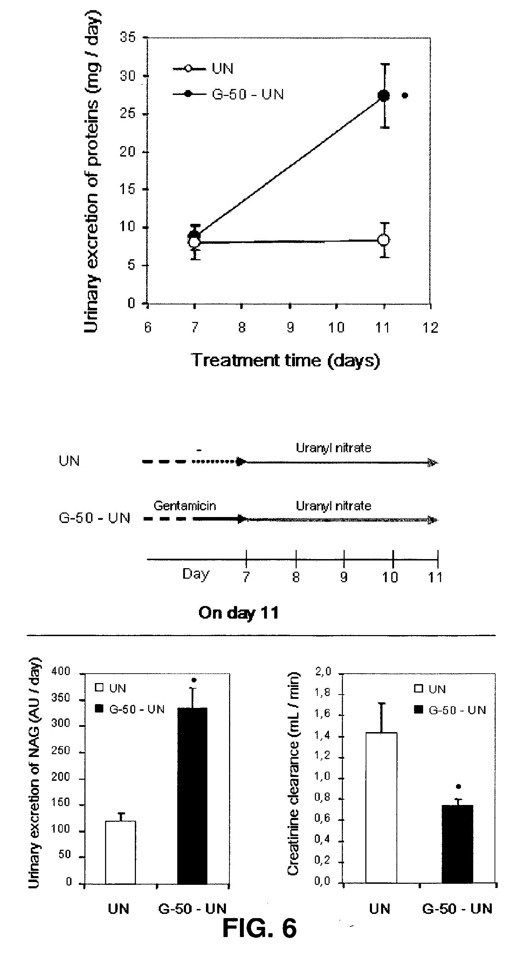

**FIG. 6****. Shows the evolution of urinary excretion of proteins** (upper left panel) in rats treated during 7 days with saline (0.9% NaCl) or gentamicin (50 mg/kg/day, G-50) during 7 days, immediately after which they received a single, subtoxic dose of uranyl nitrate (UN, 0.5 mg/kg, see lower left panel). The middle and right panels show, respectively, the urinary excretion of N-acetyl-glucosaminidase (NAG) and creatinine clearance on day 11 (6 days after UN administration) of the same animals. Data represent the mean ± standard error of 3-9 animals per condition. ● p<0.05 versus UN group; AU: arbitrary units.

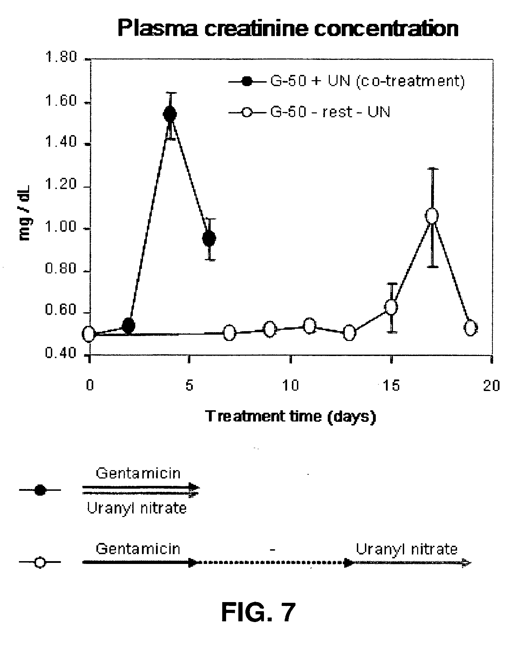

**FIG. 7****. Shows the evolution of plasma creatinine concentration** of (i) rats treated with gentamicin (50 mg/kg/day) plus a single dose of uranyl nitrate (0.5 mg/kg) administered along with the first dose of gentamicin (solid circles), and (ii) rats treated with gentamicin (50 mg/kg/day) during 7 days. After the seventh day, treatment was withdrawn for another seven days, after which a single dose of uranyl nitrate (0.5 mg/kg) was administered (open circles, see lower panel for protocol representation). Data represent the mean ± standard error of 6 animals per group.

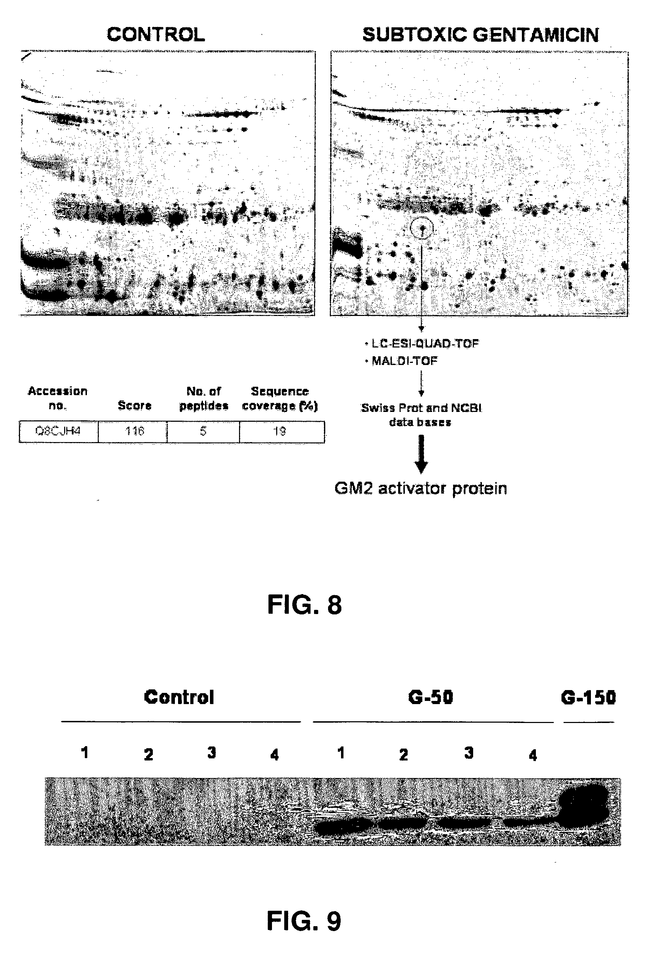

**FIG. 8****. Shows a representative 2D-electrophoresis of proteins** of urine from rats treated during 7 days with saline (0.9% NaCl, Control) or gentamicin (50 mg/kg/day). Experiments were performed with urine samples from 4 animals in each group (each sample in duplicate).
**FIG. 9****. Shows the level of GM2 activator protein in the urine** from 4 randomly selected rats treated during 7 days with saline (0.9% NaCl, Control) and four randomly selected rats treated for the same period with 50 mg/kg/day gentamicin. As a control of overt renal failure, a sample from a rat treated during 7 days with 150 mg/kg/day gentamicin is also shown.

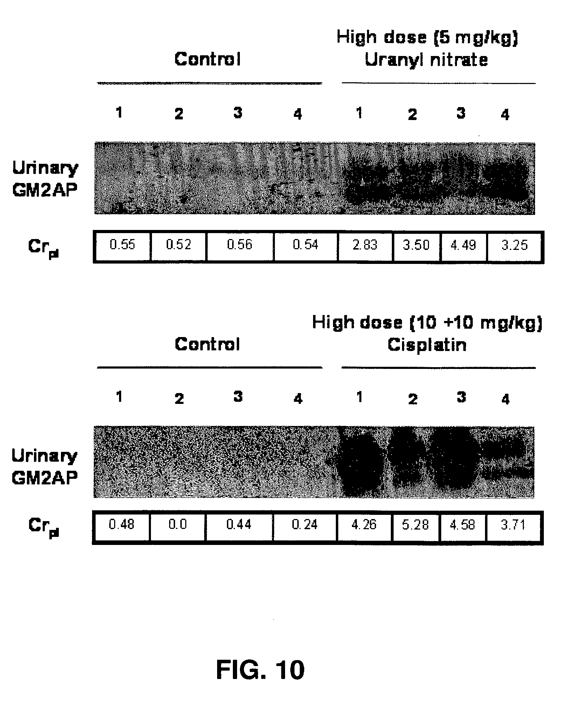

**FIG. 10****. Shows a Western blot image with the level of GM2 activator protein in the urine** from 4 rats treated with a single, nephrotoxic dose of uranyl nitrate (5 mg/kg, UN), from 4 rats treated with two doses of cisplatin (10 mg/kg) on the first and third days, or from 4 rats treated with saline (0.9% NaCl, Control). Urines were collected 4 days after treatment inception. Also, individual values of plasma creatinine concentration (Crₚₗ, mg/dL) are shown.

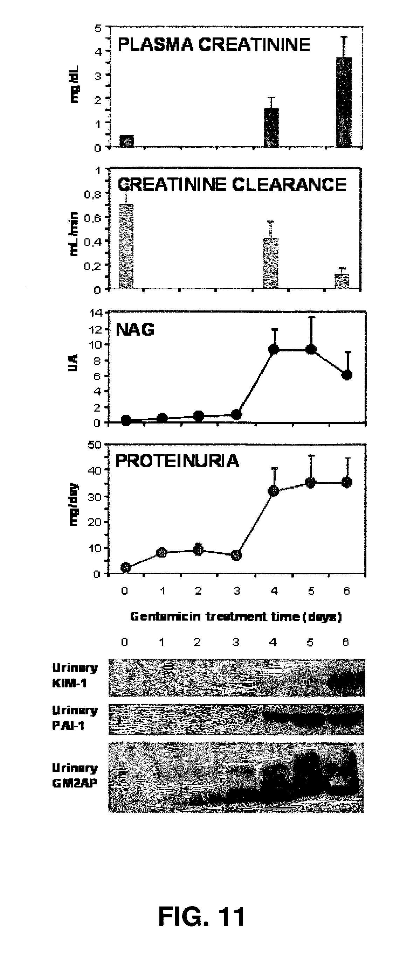

**FIG. 11****. Shows the evolution of markers of nephrotoxicity nephrotoxic effect:** plasma creatinine concentration, creatinine clearance, N-acetyl-glucosaminidase (NAG) excretion, proteinuria, and urinary levels of kidney injury molecule-1 (KIM-1), plasminogen activator inhibitor-1 (PAI-1) and GM2 activator protein (GM2AP), in rats treated during 7 days with gentamicin (150 mg/kg/day). Data are the mean ± standard error of samples from 6 animals. Images are representative of experiments performed with samples from 3 different animals.

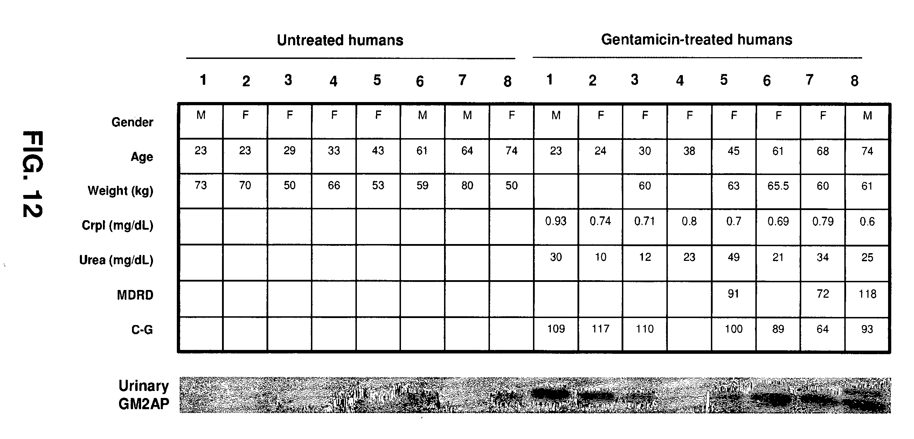

**FIG. 12****. Shows the level of GM2 activator protein (GM2AP) in the urine from 8 untreated and 8 gentamicin-treated humans.**
   Their gender, age, body weight, plasma creatinine concentration, plasma urea concentration, and the MDRD (mL/min) and Cockroft-Gault (mL/min) estimations of creatinine clearance are also shown (when known).

### EXAMPLES

The following examples provide a description, of illustrative and non-limiting character, of some of the assays and operating conditions claimed in the list given below that refers to the detection of GM2AP and its use as a biomarker to detect ARF and the risk of an acute renal failure.

### EXAMPLE 1. Methods and reagents.

### 1.1. Animals and experimental protocol.

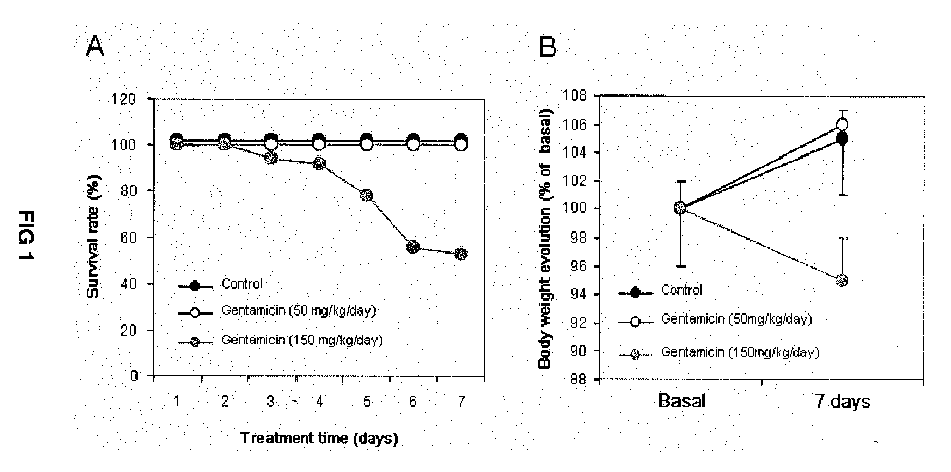

In-house bred, female Wistar rats weighing 190-230 g were divided in the following experimental groups (figure 1): (i) Control: rats treated i.p. during 7-13 days with saline (0.9% NaCl), once daily. (ii) G-50: rats treated with 50 mg/kg/day gentamicin during 6 days. (iii) G-50-NU: rats treated with 50 mg/kg/day gentamicin during 6 days, and on the seventh day treated with a single i.p. dose of 0.5 mg/kg uranyl nitrate. (iv) NU: rats treated i.p. with saline (0.9% NaCl) once daily during 6 days, and on the seventh day treated with a single i.p. dose of 0.5 mg/kg uranyl nitrate. (v) G-50 + NU: rats treated with 50 mg/kg/day gentamicin during 6 days, and on the first day treated with a single i.p. dose of 0.5 mg/kg uranyl nitrate. (vi) G-50-r-NU: rats treated with 50 mg/kg/day gentamicin during 6 days, left without treatment during one week, and then treated with a single i.p. dose of 0.5 mg/kg uranyl nitrate. (vii) G-150: rats treated with 150 mg/kg/day gentamicin during 6 days.

For the whole experiment, rats were individually allocated in metabolic cages under temperature and humidity controlled conditions. Animals were allowed to eat regular chow and drink water *ad libitum.* At different times, 24-hour urine and blood samples were obtained. Urine was collected in graduated vessels supplemented with 100 µL of 0.1% sodium azide and 1 ml oil to prevent contamination and evaporation, respectively. Subsequently, urine was cleared by centrifugation at 1,175 x g, aliquoted and stored at -80 °C. Blood was drawn from a small incision in the tail and collected in heparinised capillaries. These were immediately centrifuged at 12,000 x g and plasma was stored at -80 °C. At the end of the experiment, rats were anesthetized with sodium pentobarbital (10 mg/kg). The kidneys were perfused with heparinised saline through the abdominal aorta, and they were immediately dissected. The right kidney and one half of the left one were swiftly frozen by immersion in liquid nitrogen, and stored at -80 °C. Eventually, they were used for Western blot studies. For such a purpose, frozen tissues were homogenized under freezing conditions by hammer-smashing, until a fine powder was obtained. The powder was also kept at -80 °C. The second half of the left kidney was immersed in 3.7% paraformaldehyde at 4 °C during 14-16 hours. After it, tissue samples were processed for immunohistochemical studies.

### 1.3. In vivo perfusion of gentamicin.

Gentamicin was kindly provided by Schering-Plough. Uranyl nitrate was obtained from Sigma. Where not indicated otherwise, all other reagents were purchased from Sigma.

In-house bred, female Wistar rats weighing 190-230 g were anesthetized with sodium pentobarbital (10 mg/kg). The right jugular vein and the urinary bladder were catheterized. A single, bolus dose of 1.5 mL Gentamicin (150 mg/kg) or saline (0.9% NaCl, as control) were perfused through the jugular vein in 1.5 mL during 30 minutes. After this, urine was collected at different time points. Urine was cleared by centrifugation (as above) and kept at -80 °C for later use.

### 1.3. Renal function characterization.

Plasma and urine concentration of creatinine and blood urea nitrogen (BUN) were determined by an automated analysis system (Reflotron®, Roche Diagnostics, Barcelona, Spain) and commercial reactive strips (Roche Diagnostics, Barcelona, Spain), using 32 µL of plasma and urine. This method has a lower detection limit of 0.5 mg/dL for creatinine. Samples reading under 0.5 mg/dL creatinine were head-to-head re-assayed by the colorimetric method of Jaffé (Hervey, 1953. Nature, 171: 1125).

Creatinine clearance (Cl_{Cr}, mL/min) was calculated using the following equation: Cl_{Cr} = UF x Cᵤ / Cₚ, where UF is the 24-hour urinary flow (expressed in mL/min), Cᵤ is the urinary concentration of creatinine, and Cₚ is the plasmatic concentration of creatinine.

Urine protein concentration (mg/mL) was measured by the Bradford method (39). Daily protein excretion (mg/day) was obtained by multiplying urine protein concentration by the 24-hour urine flow (mL/day).

Urine NAG activity (arbitrary units, AU/mL), as an estimation of urine NAG concentration, was measured by a commercial enzymatic test (Roche Diagnostics, Barcelona, Spain) following the manufacturer's instructions. Urine NAG activity was converted into daily NAG excretion (AU/day) by multiplying urine NAG activity by 24-hour urine flow (mL/day).

### 1.4. Anti GM2 activator protein polyclonal serum preparation.

For the preparation of an anti-GM2AP serum, female New Zealand White rabbits were injected with a synthetic immunogen corresponding to the rat and human GM2AP partial peptide sequence SEQ ID NO: 2 plus a cysteine at the amino end to enable conjugation to the immunogenic protein blue carrier (Pierce Biotechnology; Rockford, IL) by means of the cross-linkers 4-(N-Maleimidomethyl)cyclohexane-1-carboxylic acid 3-sulfo-N-hydroxysuccinimide ester sodium salt (sulpho-SMCC) and N-Ethyl-N'-(3-dimethylaminopropyl)carbodiimide (EDC, both from Sigma-Aldhrich). Immunizations were carried out on days 1, 14, 36 and 58 with the synthetic peptide in Freund's adjuvant. On day 63, rabbits were exsanguinated under anaesthesia. The serum was purified through a HiTrap TM Protein G HP column (GE Healthcare Bio-Sciences AB; Uppsala, Sweden) and kept at -20 °C for further use.

### 1.5. Inmunohistochemical studies.

Kidneys were kept in p-formaldehyde overnight at 4 °C. Then, paraffin blocks were made and 5-µm tissue sections were cut and stained with hematoxilin and eosin. Photographs were taken under an Olympus BX51 microscope connected to an Olympus DP70 colour, digital camera.

### 1.6. Western blotting.

Protein extracts were obtained from 100 mg tissue homogenate powder, prepared by homogenizing the kidneys with a tissue mixer (Ultra-Turrax T8, IKA®-Werwe) at 4 °C in homogenization buffer (140 mM NaCl, 20 mM Tris-HCl pH=7.5, 0.5 M ethylenediaminetetraacetic acid -EDTA-, 10% glycerol, 1% Igepal CA-630, 1 µg/mL aprotinin, 1 µg/mL leupeptin, 1 µg/mL pepstatin A, 1 mM phenylmethylsulphonyl fluoride -PMSF-). Tissue homogenates were centrifuged at 22,000 g during 15 minutes at 4 °C. Supernatants were collected. Protein concentration was measured with a commercial kit (BioRad) based on the Lowry method. 50 µg total protein from tissue extracts or 20 µL cleared urine from each sample were separated by electrophoresis in 10-15% acrylamide gels (Mini Protean II system, BioRad). Immediately, proteins were electrically transferred to an Immobilon-P membrane (Millipore). After blocking the membrane to avoid unspecific binding, membranes were probed with antibodies against KIM-1 (R&D Systems), bone morphogenetic protein-7 (BMP-7, Santa Cruz Biotechnology), plasminogen activator inhibitor-1 (PAI-1, BD Biosciences), vimentin (Dako Denmark) and GM2 activator protein (GM2AP, see above).

### 1.7. Urine proteomic analysis.

Urine was concentrated and desalted by means of centrifugation-forced filtration through Amicon Ultra 5 K cut-off columns (Millipore). Protein concentration was determined by the Bradford method. Urine proteins were precipitated with the Clean-Up kit (GE Healthcare) according to the manufacturer's instructions. 100 mg of protein from each sample were rehydrated in 7 M urea, 2 M thiourea, 4% (w/v) Chaps, 0.5% ampholytes pH 4-7 or 4.5-5.5), 50 mM dithiothreitol (DTT) and bromophenol blue, and isoelectrically focused (500-8,000 V) through 18-cm long immobilized pH gradient (IPG) strips, pH 4-7 or 4.5-5.5 (GE Healthcare, Madrid, Spain), using an IPGphor apparatus (GE Healthcare). IPG strips were pre-equilibrated during 15 minutes in equilibration buffer [50 mM Tris-HCl pH=8.8, 6 M urea, 30% (v/v) glycerol, 2% (w/v) sodium dodecylsulphate (SDS), 0.01% (w/v) bromophenol blue] containing 1% (w/v) DTT, and another 15 minutes in equilibration buffer containing 2.5% (w/v) iodoacetamide. Then, IPG strips were transferred to 18-cm long, 12% acrylamide gels and separated by electrophoresis with a SE 600 Ruby apparatus (GE Healthcare). Gels were fixed overnight in 30% ethanol, 10% acetic acid and silver stained with a commercial kit (GE Healthcare). Unless otherwise indicated, all reagents were from Sigma.

For visualization and analysis, stained gels were scanned (Image Scanner, GE Healthcare, Madrid, Spain), and processed and statistically analyzed with the Image Master 2D Platinum 6.0 software (GE Healthcare, Madrid, Spain). Spot discrimination was done with the following parameters: (i) smooth factor: 2; (ii) minimal area: 5 pixels; (iii) saliency: 100. Analysis was visually corrected for artefact elimination. For each individual spot, background was subtracted and individual intensity volume was normalized by total intensity volume (all-spot intensity). For comparison of the same spot among gels, a minimum of a twofold intensity difference was established to consider a differential expression. The bands and spots of interest from 1 D and 2D separations (respectively) were cut off the gels. Each gel piece was dehydrated in acetonitrile and this was evaporated with a vacuum pump and resuspended in NH₄HCO₃. 2D-CF fractions were also vacuum evaporated and residues resuspended in NH₄HCO₃. Hence on, samples from 1 D, 2D and 2D-CF were treated identically. They were reduced with 10 mM DTT in 50 mM NH₄HCO₃ at 56 °C, and alkylated with iodoacetamide in 50 mM NH₄HCO₃. Then the proteins were in-gel digested into peptides with porcine trypsin (Promega) during 30 minutes at 4 °C. Subsequently, peptides were extracted with 0.5% (v/v) trifluoroacetic acid (TFA). The solution was vacuum evaporated and peptides were dissolved in 0.1% (v/v) formic acid under sonication. Peptide-containing solutions were injected in a LC-ESI-QUAD-TOF mass spectrophotometer QSTAR XL (Applied Biosystems) with an1100 micro HPLC (Agilent). A wide pore 150x0.32 mm (5 µm) Supelco column (Discovery BIO) was used at a flow rate of 7 µL/min. MS/MS spectra were obtained. Protein identification was performed with the MASCOT software against non redundant protein sequence databases (Swiss Prot and NCBI). Mass tolerance was set at 50 ppm, MS/MS tolerance was 0.5 Da, and the taxonomic status was Rattus. Only significant hits, as identified by MASCOT probability analysis, were considered and at least one peptide match with ion score above 20 was set as the threshold of acceptance. In some instances where the LC-ESI-QUAD-TOF method did not yield an unambiguous protein identification, or in randomly selected spots (for confirmation), proteins where identified with an Ultraflex I MALDI-TOF mass spectrophotometer (Bruker Daltonics) by the Proteomic Service of the *Centro de Investigación del Cancer of the Universidad de Salamanca-CSIC (Salamanca, Spain).*

Urine samples from human hospitalized individuals under a gentamicin regime already in course for 2 or 3 days were obtained from the *Hospital Universitario de Salamanca.* As controls, the urine from age- and sex-matched individuals not receiving gentamicin was used.

### EXAMPLE 2. Results.

The body of results presented herein is intended to demonstrate that: (i) a regime of 6 daily doses of 50 mg/kg gentamicin does not, by any parameter studied, induce any renal injury or dysfunction; (ii) despite this, this sub-nephrotoxic regime predisposes rats to the development of an ARF by lowering the toxicity threshold of a second nephrotoxin; as a consequence, a sub-nephrotoxic dose of this second nephrotoxin triggers an ARF in gentamicin-predisposed rats, but not in untreated rats; (iii) through a differential proteomic approach on the urine from G-50 animals (compared to controls), a urinary protein (i.e. ganglioside GM2 activator protein, GM2AP) was identified, which detects this condition; (iv) this new urinary biomarker can be used not only for the detection of this hitherto hidden, predisposing condition, but also as a very early marker of gentamicin-induced acute renal injury.

### 2. 1. Selection of a gentamicin regime with no nephrotoxic effects.

In pilot studies, the dose-to-nephrotoxic effect relationship of gentamicin was titrated in Wistar rats (data not shown). As a result, it was concluded that a regime of 6 daily doses of 50 mg/kg/day (G-50) produced no signs of nephrotoxicity, whereas a regime of 6 daily doses of 150 mg/kg/day (G-150) caused a marked renal failure that served as a positive control for all our measurements. Figures 1 thru 4 show a detailed characterization of the effect of the G-50 and G-150 regimes on renal function and morphology, compared to control rats (C) receiving saline. These experiments clearly demonstrate that the G-50 regime exerts no deleterious effect on the general health status of the animals, and no single specific sign of nephrotoxicity, as measured by (i) parameters used in the clinical practice, and (ii) others that more deeply characterize renal status.

FIG. 1 indicates that animals under G-50 show a survival rate and an evolution of body weight similar to those of control animals, suggesting that toxicity was not significant. By contrast, animals under G-150 showed a death rate of about a 50%, and a body weight loss indicative of serious health deterioration. After treatment withdrawal (day 8 and on), no animal from any of the groups died.

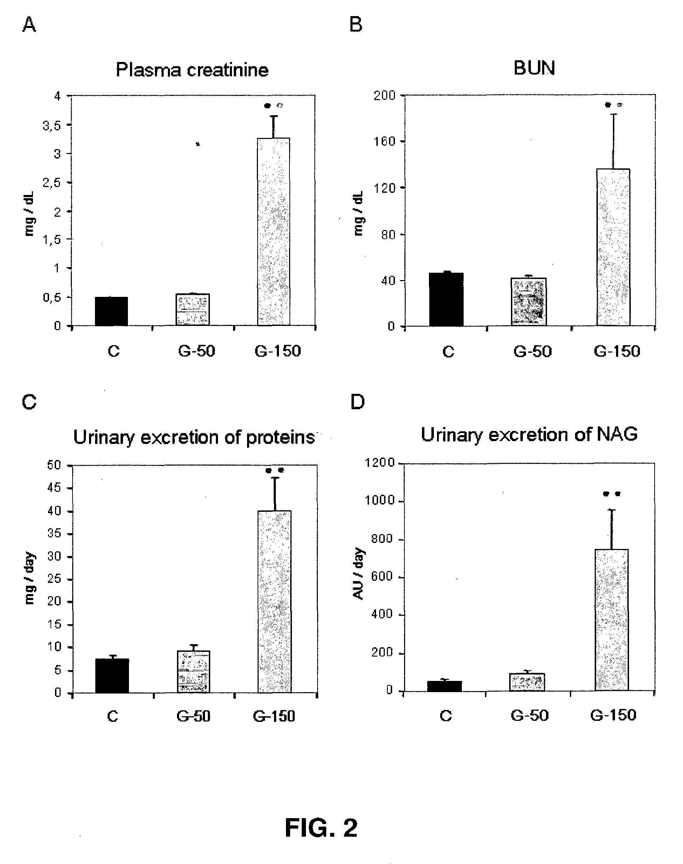

Data in FIG. 2 indicate that, compared to control rats, in G-50 rats clinically used parameters associated to GFR status (i.e. plasma creatinine and BUN concentrations) were not modified, whereas in the G-150 group they were profoundly altered (panels A and B). In gentamicin-induced renal damage, proteinuria is considered to be mostly from tubular origin (ref). Accordingly, panel C shows that G-50 animals show no proteinuria (compared to control ones), whereas G-150 animals have an overt proteinuria. This is consistent with data from panel D, where urinary NAG excretion, a very sensitive marker of tubular damage (ref), is not modified in G-50 and very increased in G-150. These data indicate that no renal function alteration is observed upon G-50 regime, whereas an evident acute renal failure occurs as a result of G-150 treatment. This latter is consistent with a 50% death rate and health deterioration observed in these animals.

Images in FIG. 3 indicate that the G-150 regime inflicts an intensive injury to renal parenchyma, mainly characterized by a massive tubular necrosis (panel C). However, no gross tissue alterations were detected in the G-50 group, compared to the control group.

Furthermore, Western blot analysis of renal tissue homogenates (FIG. 4) show a clear increase of markers related to tissue injury and repair, such as PAI-1, KIM-1 and vimentin, in the G-150 group. However, no increment of these markers was seen in samples from the G-50, compared to those from the control group.

Together with the histological results depicted in FIG. 3, tissue marker analysis reinforces the idea that the G-50 regime causes no tissue injury to the kidneys.

### 2.2. A sub-toxic regime with gentamicin predisposes to the development of an acute renal failure.

The body of results in this section is intended to demonstrate that the sub-toxic G-50 regime predisposes rats to the development of an acute renal injury when subject to a second potential nephrotoxin (event at sub-toxic doses for untreated rats). This predisposition induced by sub-toxic gentamicin occurs during gentamicin treatment and continues at least during another week after treatment withdrawal.

FIG. 5 shows that when rats are subject to the nephrotoxin uranyl nitrate (UN, 0.5 mg/kg), their plasma concentration of creatinine and BUN increases only if they have been previously subject to the sub-toxic regime with gentamicin (G-50). No alterations in these parameters are observed in those rats receiving only gentamicin (G-50), UN or saline (as control).

Moreover, only rats previously predisposed by gentamicin show proteinuria (FIG. 6, left panel), increased NAG excretion and decreased creatinine clearance (FIG. 6, right panel). These data further reinforce the information from FIG. 5, and altogether support the predisposing action of gentamicin.

Interestingly, the predisposing effect of gentamicin is effective not only immediately after gentamicin withdrawal, but also one week after it, and even during gentamicin treatment. This is demonstrated by data in FIG. 7, where rats were subject to UN in parallel to gentamicin (black circle), or one week after gentamicin withdrawal (open circle).

### 2.3. Identification of urinary markers of gentamicin-induced predisposition to acute renal failure through differential proteomics.

The state-of-the-art clinical technology is not capable of detecting or diagnosing the predisposing action of gentamicin, because no parameter is altered under those conditions. With the objective of finding new urinary markers associated to this effect of gentamicin that might be used to diagnose the condition, we set out a differential proteomic approach with urine from control and G-50 animals. The experiments (FIG. 8) identified GM2AP as absent in control urine and highly expressed in G-50 urines.

### 2.4. Characterization and validation of GM2AP as a urinary marker of gentamicin-induced predisposition to acute renal failure.

By means of a polyclonal antibody generated against rat and human GM2AP we further confirmed the differential level of this protein in the urine of control and G-50 (FIG. 9). Our results also demonstrate that this protein is increased in the urine of G-150 rats with an overt renal failure (FIG. 9, and see below, FIG. 11 for further details).

### 2.5. GM2AP after other nephrotoxic drugs (UN and cisplatin).

FIG. 10 demonstrates that GM2AP may serve also for the detection of the overt renal injury inflicted by other nephrotoxic agents, such as UN (upper panel) and cisplatin (lower panel). It can be observed that rats undergoing an ARF either by UN or cisplatin, as demonstrated by their plasma creatinine concentration show high levels of urinary GM2AP, whereas control rats show very little or undetectable amounts of this marker.

From all the preceding data we can conclude that GM2AP may serve to diagnose: 1. The predisposition to ARF induced by sub-toxic gentamicin. This constitutes a breakthrough in ARF diagnosis, since we might be now enabled to detected a condition that might silently predisposed gentamicin-treated patients to more easily develop acute renal injury by exposure to other agents that in normal conditions would not cause any renal effects. Furthermore, the next thing to be studied is whether this novel marker in ARF also serves to detect or diagnose the potential predisposing effect that other drugs might induce at sub-toxic doses.

### 2.6. Characterization and validation of GM2AP as a very early marker of acute renal failure (or specific forms of ARF).

In order to study how early GM2AP detects renal injury, a series of time course experiments on the evolution of different markers upon treatment with the G-150 regime where carried out. As can be observed in FIG. 11, clinical parameters such as creatinine clearance, plasma creatinine and BUN concentration, proteinuria, and NAG excretion were increase by day 4 after the inception of gentamicin treatment. Moreover, urinary levels of KIM-1 and PAI-1 were also increased by the day 4. Interestingly, urinary levels of GM2AP started to increase as early as at day 1 after gentamicin onset.

This clearly indicates that GM2AP might be developed as a very early marker of gentamicin-induced acute renal injury, and maybe of acute renal injury in general.

### 2.7. Characterization of GM2AP in the urine of humans treated with gentamicin.

Finally, we tested whether GM2AP levels are increased in the urine of patients treated with gentamicin. FIG 12 shows anthropometric data, plasma creatinine and BUN concentrations, and GFR estimated by the MDRD or Cockroft-Gault equations of patients under a gentamicin regime, and anthropometric data from healthy, untreated controls. Renal function of gentamicin-treated patients included is within normal status. However, the urinary level of GM2AP, as determined by Western blot, is clearly higher in patients treated with gentamicin, similarly to what is found in experimental animals. This, along with all the data collected in rats suggests that urinary levels of GM2AP should be validated as clinical markers of predisposition to acute renal injury in humans.

## Claims

1. A method to provide data for determining the risk in an individual of developing acute renal failure (ARF), or for determining ARF, comprising detecting and/or quantifying a protein at least 70% or 100% identical to SEQ ID NO: 1, in a biological sample isolated from an individual (subject), preferably the individual is a human.

2. The method according to claim 1, wherein in addition it comprises comparing the data obtained with standard values to find any significant deviation.

3. The method according to claim 2, wherein in addition it comprises the attribution of the significant deviation to the risk of developing ARF, or to developing of an ARF, in the individual.

4. The method according to any of claims 1 to 3, wherein the biological sample is a bodily fluid, preferably the bodily fluid is urine.

5. The method according to any of claims 1 to 4, wherein the protein is detected and/or quantified by means of electrophoresis, immunoassay, chromatography and/or microarray technologies.

6. The method according to any of claims 1 to 5, wherein the risk of developing ARF, or the ARF, is due to the administration of or exposure to at least one nephrotoxic agent.

7. The method according to claim 6, wherein the nephrotoxic agent is an aminoglycoside antibiotic, preferably the aminoglycoside antibiotic is gentamicin.

8. The method according to any of claims 6 or 7, wherein the protein is detected from 12 hours or from 24 hours after the beginning of the administration of or exposure to the nephrotoxic agent.

9. A method of predicting the progression of an ARF due to the administration of or exposure to at least one nephrotoxic agent, comprising:
a. Determining a first concentration of a protein at least 70% or 100% identical to SEQ ID NO: 1, in a bodily fluid, preferably the bodily fluid is urine, isolated from an individual exposed or not to the nephrotoxic agent, preferably the individual is a human,
b. determining a second concentration of the protein from step (a) in a bodily fluid isolated from the individual after determining the first concentration of the protein in exposed individual, or after initiation of administration or exposure to the nephrotoxic agent in not exposed individual, and
c. comparing said second concentration with said first concentration finding any significant deviation.

10. The method according to claim 9, wherein the nephrotoxic agent is an aminoglycoside antibiotic, preferably the aminoglycoside antibiotic is gentamicin.

11. A use of a protein at least 70% or 100% identical to SEQ ID NO: 1, as biomarker for determining the risk of developing ARF, or for determining ARF, or for predicting the progression of an ARF.

12. The use of the protein according to claim 11, wherein the risk of developing ARF, or the ARF, or the progression of an ARF is due to the administration of at least one nephrotoxic agent.

13. A use of a kit to provide data for determining the risk of developing ARF, or for determining ARF, or for predicting the progression of an ARF, in a sample isolated from a individual, wherein the kit comprises one or more specific antibodies to detect a protein at least 70% or 100% identical to SEQ ID NO: 1.

14. The use of the kit according to claim 13, wherein the specific antibodies are attached to a solid support.

15. The use of the kit according to any of claims 13 to 14, wherein the specific antibodies are used to detect the protein or the fragment of the protein with SEQ ID NO: 2.

## Patentansprüche

1. Verfahren zur Bereitstellung von Daten zur Bestimmung des Risikos für die Entwicklung eines akuten Nierenversagens (ANV) oder zur Bestimmung des ANV in einem Individuum, das den Nachweis und/oder die Quantifizierung eines Proteins, das zu mindestens 70 % oder 100 % identisch mit der SEQ ID NO: 1 ist, in einer aus einem Individuum (Subjekt) isolierten biologischen Probe umfasst, wobei das Individuum vorzugsweise ein Mensch ist.

2. Verfahren nach Anspruch 1, wobei es zusätzlich den Vergleich der erhaltenen Daten mit Standardwerten umfasst, um jegliche signifikante Abweichung festzustellen.

3. Verfahren nach Anspruch 2, wobei es zusätzlich die Zuordnung der signifikanten Abweichung dem Risiko für die Entwicklung eines ANV oder der Entwicklung eines ANV in einem Individuum umfasst.

4. Verfahren nach einem der Ansprüche 1 bis 3, wobei die biologische Probe eine Körperflüssigkeit ist, wobei die Körperflüssigkeit vorzugsweise Urin ist.

5. Verfahren nach einem der Ansprüche 1 bis 4, wobei das Protein durch Elektrophorese, Immunassay, Chromatographie und/oder Mikroarray-Technologien nachgewiesen und/oder quantifiziert wird.

6. Verfahren nach einem der Ansprüche 1 bis 5, wobei das Risiko für die Entwicklung eines ANV oder das ANV auf der Verabreichung oder Einwirkung mindestens eines nierentoxischen Mittels beruht.

7. Verfahren nach Anspruch 6, wobei das nierentoxische Mittel ein Aminoglykosid-Antibiotikum ist, wobei das Aminoglykosid-Antibiotikum vorzugsweise Gentamicin ist.

8. Verfahren nach einem der Ansprüche 6 oder 7, wobei das Protein ab 12 Stunden oder ab 24 Stunden nachdem mit der Verabreichung oder der Einwirkung des nierentoxischen Mittels begonnen wurde nachgewiesen wird.

9. Verfahren zur Vorhersage des Verlaufs eines ANV aufgrund der Verabreichung oder Einwirkung mindestens eines nierentoxischen Mittels, das
a. die Bestimmung einer ersten Konzentration eines Proteins, das zu mindestens 70 % oder 100 % identisch mit der SEQ ID NO: 1 ist, in einer Körperflüssigkeit, wobei die Körperflüssigkeit vorzugsweise Urin ist, die aus einem dem nierentoxischen Mittel ausgesetzten oder nicht ausgesetzten Individuum isoliert wurde, wobei das Individuum vorzugsweise ein Mensch ist,
b. die Bestimmung einer zweiten Konzentration des Proteins aus Schritt (a) in einer Körperflüssigkeit, die aus dem Individuum nach der Bestimmung der ersten Konzentration des Proteins im ausgesetzten Individuum oder nach Beginn der Verabreichung oder Einwirkung des nierentoxischen Mittels in einem nicht ausgesetzten Individuum isoliert wurde, und
c. den Vergleich der besagten zweiten Konzentration mit besagter ersten Konzentration, um jegliche signifikante Abweichung festzustellen,
umfasst.

10. Verfahren nach Anspruch 9, wobei das nierentoxische Mittel ein Aminoglykosid-Antibiotikum ist, wobei das Aminoglykosid-Antibiotikum vorzugsweise Gentamicin ist.

11. Verwendung eines Proteins, das zu mindestens 70 % oder 100 % identisch mit der SEQ ID NO: 1 ist, als Biomarker zur Bestimmung des Risikos für die Entwicklung eines ANV oder zur Bestimmung eines ANV oder zur Vorhersage des Verlaufs eines ANV.

12. Verwendung des Proteins nach Anspruch 11, wobei das Risiko für die Entwicklung eines ANV oder das ANV oder der Verlauf eines ANV auf der Verabreichung mindestens eines nierentoxischen Mittels beruht.

13. Verwendung eines Kits für die Bereitstellung von Daten zur Bestimmung des Risikos für die Entwicklung eines ANV oder zur Bestimmung eines ANV oder zur Vorhersage des Verlaufs eines ANV in einer aus einem Individuum isolierten Probe, wobei der Kit einen oder mehrere spezifische Antikörper zum Nachweis eines Proteins, das zu mindestens 70 % oder 100 % identisch mit der SEQ ID NO: 1 ist, umfasst.

14. Verwendung des Kits nach Anspruch 13, wobei die spezifischen Antikörper an einen festen Träger gebunden sind.

15. Verwendung des Kits nach einem der Ansprüche 13 bis 14, wobei die spezifischen Antikörper zum Nachweis des Proteins oder des Proteinfragments der SEQ ID NO: 2 verwendet werden.

## Revendications

1. Un procédé pour fournir des données afin de déterminer le risque que présente un individu de développer une insuffisance rénale aiguë (IRA), ou afin de déterminer une IRA, comprenant la détection et/ou la quantification d'une protéine à 70% ou à 100% identique à SEQ ID NO: 1, dans un échantillon biologique isolé chez un individu (sujet), l'individu étant de préférence un humain.

2. Le procédé selon la revendication 1, dans lequel est comprise en outre la comparaison des données obtenues avec des valeurs standard afin de repérer tout écart significatif.

3. Le procédé selon la revendication 2, dans lequel est comprise en outre l'attribution de l'écart significatif au risque de développer une IRA, ou au développement d'une IRA, chez l'individu.

4. Le procédé selon n'importe laquelle des revendications 1 à 3, dans lequel l'échantillon biologique est un fluide corporel, le fluide corporel étant de préférence de l'urine.

5. Le procédé selon n'importe laquelle des revendications 1 à 4, dans lequel la protéine est détectée et/ou quantifiée par électrophorèse, immuno-essai, chromatographie et/ou des technologies avec biopuces.

6. Le procédé selon n'importe laquelle des revendications 1 à 5, dans lequel le risque de développer une IRA, ou l'IRA, est dû à l'administration de ou à l'exposition à au moins un agent néphrotoxique.

7. Le procédé selon la revendication 6, dans lequel l'agent néphrotoxique est un antibiotique aminoglycoside, l'antibiotique aminoglycoside étant de préférence la gentamicine.

8. Le procédé selon n'importe laquelle des revendications 6 ou 7, dans lequel la protéine est détectée à partir de 12 heures ou à partir de 24 heures après le commencement de l'administration ou de l'exposition de l'agent néphrotoxique.

9. Un procédé pour prévoir la progression d'une IRA due à l'administration ou l'exposition à au moins un agent néphrotoxique, consistant à :
a. Déterminer une première concentration d'une protéine identique au moins à 70% ou à 100% à SEQ ID NO: 1, dans un fluide corporel, le fluide corporel étant de préférence de l'urine, isolé chez un individu exposé ou non à l'agent néphrotoxique, l'individu étant de préférence un humain.
b. Déterminer une seconde concentration de la protéine de l'étape (a) dans un fluide corporel isolé chez l'individu après avoir déterminé la première concentration de la protéine chez l'individu exposé, ou après avoir initié l'administration ou l'exposition à l'agent néphrotoxique chez l'individu non exposé, et
c. comparer ladite seconde concentration à ladite première concentration en repérant tout écart significatif.

10. Le procédé selon la revendication 9, dans lequel l'agent néphrotoxique est un antibiotique aminoglycoside, l'antibiotique aminoglycoside étant de préférence la gentamicine.

11. Une utilisation d'une protéine identique au moins à 70% ou à 100% à SEQ ID NO: 1, comme biomarqueur pour déterminer le risque de développement d'une IRA, ou pour déterminer une IRA, ou pour prévoir la progression d'une IRA.

12. L'utilisation de la protéine selon la revendication 11, dans laquelle le risque de développement d'une IRA, ou l'IRA, ou la progression d'une IRA est dû à l'administration d'au moins un agent néphrotoxique.

13. Une utilisation d'un kit pour fournir des données afin de déterminer le risque de développement d'une IRA, ou pour déterminer une IRA, ou pour prévoir la progression d'une IRA, dans un échantillon isolé chez un individu, dans laquelle le kit comprend un ou plusieurs anticorps spécifiques pour détecter une protéine identique au moins à 70% ou à 100% à SEQ ID NO: 1.

14. L'utilisation du kit selon la revendication 13, dans laquelle les anticorps spécifiques sont fixés à un support solide.

15. L'utilisation du kit selon n'importe laquelle des revendications 13 à 14, dans laquelle les anticorps spécifiques sont utilisés pour détecter la protéine ou le fragment de la protéine avec SEQ ID NO: 2.
